# EUROPÄISCHE PATENTANMELDUNG

(11) **EP 3 895 756 A1**
(43) Veröffentlichungstag der Anmeldung: **20.10.2021**
(21) Anmeldenummer: 21165356.3
(22) Anmeldetag: 27.04.2017
(51) Int. Cl.: A61M 60/50, A61M 1/16, A61M 1/36

(54) **SYSTEM FÜR DIE EXTRAKORPORALE MEMBRANOXY GENIERUNG MIT EINER BLUTPUMPE UND EINEM OXYGENATOR**

(30) Priorität: 18.05.2016 DE 102016006013
(62) Teilanmeldung aus: 17731799.7
(71) Anmelder: Xenios AG, 74076 Heilbronn (DE)
(72) Erfinder: Simundic, Ivo, 73240 Wendlingen (DE)
(74) Vertreter: Graf von Stosch Patentanwaltsgesellschaft mbH

(57) **Zusammenfassung**

Bei einem System für die extrakorporale Membranoxygenierung mit einer Blutpumpe und einem Oxygenator weist der Oxygenator in einem Gehäuse gestapelte Matten mit Fasern auf, die parallel zueinander angeordnet sind, wobei die Blutpumpe eine Steuerung aufweist, die eine kontinuierliche Variation des Strömungsvolumens über der Zeit ermöglicht.

## Beschreibung

Die Erfindung betrifft ein System für die extrakorporale Membranoxygenierung mit einer Blutpumpe und einem Oxygenator, wobei die Blutpumpe eine Steuerung aufweist, die eine kontinuierliche Variation des Strömungsvolumens über der Zeit ermöglicht.

Unter einer kontinuierlichen Variation des Strömungsvolumens über der Zeit wird eine Veränderung des Strömungsvolumens verstanden, die nicht auf ein Anschalten und Ausschalten der Blutpumpe zurückzuführen ist. Außerdem sind hierunter nicht Strömungsvolumenveränderungen zu verstehen, die auf die Art der Blutpumpe zurückzuführen sind, wie beispielsweise bei einer Rollerpumpe oder auch bei einer Zentrifugalpumpe, die bauartbedingt keinen absolut kontinuierlichen Strömungsfluss erzeugen.

Die kontinuierliche Variation des Strömungsvolumens über der Zeit resultiert aus einer steuerungstechnischen Überlagerung der Ansteuerung der Pumpe durch eine Variation des Strömungsvolumens in Folge eines äußeres Einflusses auf die Ansteuerung der Pumpe. Hierdurch kann beispielsweise ein pulsatiler Strom entstehen, der wie eine Sinuskurve oder ein Rechteckimpuls über längere Zeit schwingend eine Variation des Strömungsvolumens bewirkt. Es können jedoch auch aufgrund von Messwerten, abgestimmt auf die aktuelle Situation des Körpers des Patienten, Volumenstromveränderungen vorgenommen werden, die wiederum vorzugsweise zyklisch in Abstimmung mit dem Herzschlag einen definierten Volumenstrom bzw. eine definierte Variation des Volumenstroms vorgeben.

Über die Variation des Strömungsvolumens kann der Betrieb des Systems für die extrakorporale Membranoxygenierung an die aktuelle Situation des Herzens des Patienten angepasst werden. Es können vom Arzt vorgegebene Abweichungen von einem kontinuierlichen Volumenstrom insbesondere im Rahmen einer Veränderung von Amplitude und/oder Wellenlänge umgesetzt werden und eine pulsatile Ansteuerung der Blutpumpe kann auch dazu genutzt werden, im System festhängende Luftblasen zu lösen, um das System schneller zu entlüften.

Ein derartiges System ist beispielsweise aus der DE 10 2013 012 433 A1 bekannt.

Für diese Systeme wurde als Oxygenator eine Einrichtung verwendet, wie sie in der EP 0 765 689 beschrieben ist. Dies ermöglicht es auf einfache Art und Weise, das Blut mit Sauerstoff anzureichern und zu temperieren. Außerdem federn diese Oxygenatoren an der Blutpumpe entstehende Druckspitzen ab.

Ein derartiges System steht in der Regel über Kanülen mit dem Blutkreislauf eines Menschen und insbesondere mit dessen Herz in Verbindung. Dabei soll die Pulsatilität am Ausgang einer im Herzen angeordneten Kanüle einer vom Arzt genau vorgegebenen Pulsatilität entsprechen. Da es aufwändig wäre, die Pulsatilität am Ausgang der Kanüle im Herzen zu messen, werden die Strömungswerte des Blutes am Pumpenausgang gemessen und der Arzt schätzt mit Bezug auf das verwendetes System aus Blutpumpe Oxygenator und Kanülen beim Einstellen der Pumpensteuerung ab, welche Pulsatilität am Ausgang der Kanüle im Herzen zu erwarten ist.

Der Erfindung liegt die Aufgabe zu Grunde, ein derartiges System weiter zu entwickeln.

Nach einem ersten erfindungsgemäßen Aspekt weist der Oxygenator des Systems in einem Gehäuse gestapelte Matten mit Fasern auf, die parallel zueinander angeordnet sind.

Ein derartiger Membranoxygenator kann quer zu den gestapelten Matten von Blut durchströmt werden und er weist dann einen besonders geringen Druckabfall auf. Dies führt dazu, dass ein am Oxygenator gemessener Wert zum Strömungsverhalten des Blutes weitgehend dem Wert am Ausgang der Kanüle im Herzen entspricht.

Da der pulsatile Blutstrom durch den Oxygenator kaum verändert wird, entspricht die Pulsatilität vor dem Oxygenator fast genau der Pulsatilität nach dem Oxygenator. Dies ermöglicht einen pulsatilen Blutstrom am Ausgang des Systems und gleichzeitig eine schonende Beschleunigung des Blutes an der Pumpe innerhalb des Systems.

Besonders vorteilhaft ist eine Ausführungsvariante, bei der die Steuerung mit einem EKG in Verbindung steht. Dies ermöglicht es, die mit dem EKG gemessenen Werte direkt oder in einer verarbeiteten Form zur Steuerung der Blutpumpe zu verwenden. Dadurch kann im einfachsten Fall der vom EKG gemessene Zyklus zur Steuerung der Pumpe verwendet werden. Eine Verrechnung der EKG-Werte ermöglicht es darüber hinaus, systematisch steuernd einzuwirken, um über die Blutpumpe auf das Strömungsverhalten am Herz zu bestimmen.

Ein weiterer Aspekt der Erfindung betrifft die Ausbildung des Rotors. Besonders vorteilhaft hat sich eine Blutpumpe erwiesen, die einen Rotor aufweist, dessen äußerer Durchmesser kleiner als 4 cm, bevorzugt kleiner als 3,5 cm ist, und einen Durchmesser von mehr als 1 cm aufweist. Ein derart kleiner Rotor führt zu einem kleinen Pumpengehäuse. Außerdem sinkt bei einer Verkleinerung des Rotordurchmessers dessen Massenträgheitsmoment. Dies ermöglicht es, besonders schnell die Rotorgeschwindigkeit zu verändern, um ein spezielles Strömungsprofil des Volumenstroms über der Zeit zu erreichen. Ein größerer Rotordurchmesser könnte einen größeren Druck aufbauen, um den Widerstand des Systems zu überwinden. Derartig große Rotoren werden aber zwangsweise träge und unpräzise. Die vorgeschlagenen Rotordurchmesser ermöglichen durch das spezielle Massenträgheitsmoment ein optimales Timing für den Puls. Dies ist für die Verwendung beliebiger Oxygenatoren vorteilhaft. Insbesondere in Verbindung mit in einem Gehäuse gestapelten Matten mit Fasern, die parallel zueinander angeordnet sind, wird dieses Strömungsprofil weitgehend unverändert während des Durchflusses des Blutes durch das System bis zum Ausgang der Kanüle im Herzen aufrecht erhalten.

Daher wird vorgeschlagen, dass die Blutpumpe einen Rotor aufweist, dessen Massenträgheitsmoment kleiner als 5000 g/mm² und vorzugsweise kleiner als 1000 g/mm² ist. Um die Funktion der Blutpumpe zu gewährleisten, ist ihr Massenträgheitsmoment größer als 200 g/mm². Positive Ergebnisse wurden mit einem Rotor erzielt, dessen Massenträgheitsmoment bei etwa 660 g/mm² liegt.

Vorteilhaft ist es, wenn die Blutpumpe einen Rotor aufweist, der über Magnete mit einem Antrieb in Verbindung steht, der den Rotor um eine Achse dreht, wobei die Magnete in einem mittleren Abstand von 5 bis 10 mm von der Achse entfernt angeordnet sind. Dies führt einerseits zu einer optimierten Funktion der Magnete, die das Massenträgheitsmoment des Rotors nur geringfügig erhöhen sollten, und erlaubt andererseits eine gute Kopplung zwischen Rotor und Motor über die Magnete.

Als Blutpumpe eignen sich vor allem Blutpumpen, die nur einen geringen radialen Strömungsanteil aufweisen. Es wird daher vorgeschlagen, dass die Blutpumpe einen Rotor aufweist, der einen axialen Strömungsanteil bewirkt. Dies sind insbesondere Axial- oder Diagonalpumpen.

Eine besonders vorteilhafte Ausgestaltung des Oxygenators sieht vor, dass der Querschnitt des Blutzulaufs vor der angeströmten Matte vergrößert ist, um die Strömungsgeschwindigkeit des Blutes zu reduzieren. Dies ermöglicht es, auf dem Weg zum Oxygenator mit einem kleinen Leitungsdurchmesser und hohen Strömungsgeschwindigkeiten Blut zum Oxygenator zu fördern und kurz vor der angeströmten Matte die Geschwindigkeit des Blutes zu reduzieren, indem dort der für die Strömung zur Verfügung stehende Querschnitt deutlich vergrößert wird.

Entsprechend kann der Querschnitt des Blutzulaufes auch nach der angeströmten Matte verkleinert werden, um die Strömungsgeschwindigkeit des Blutes zu erhöhen.

Eine spezielle Ausführungsform, die einen weiteren Aspekt der Erfindung darstellt, sieht vor, dass zwischen Pumpe und Oxygenator eine Druckabfederung angeordnet ist. Damit können Druckspitzen abgefangen werden, um die mechanische Belastung von Pumpe und/oder Oxygenator zu reduzieren. Die Druckabfederung ist vorzugsweise in Strömungsrichtung zwischen Pumpe und Oxygenator angeordnet. Als Druckabfederung können ein Ausgleichsgefäß mit Luftpolster oder eine Leitung aus flexiblem Material dienen. Eine derartige flexible Leitung kann kurzzeitig aufgeweitet werden, um durch die Vergrößerung des Querschnitts eine Druckspitze abzufangen.

Eine Druckabfederung kann jedoch auch durch eine flexible oder flexibel gelagerte Blutverteilerplatte erzielt werden. Eine derartige Blutverteilerplatte hat den zusätzlichen Vorteil, dass eine direkte Anströmung auf die Hohlfasern vermieden wird.

Es wird daher weiterhin vorgeschlagen, dass die Druckabfederung ein Ausgleichsgefäß mit einem Gaspolster aufweist oder eine Leitung mit mindestens einem flexiblen Wandbereich.

Eine andere Möglichkeit der Abfederung von Druckstößen ist das bewegliche Halten der Matten des Oxygenators in einem Rahmen. Insbesondere wenn die Matten des Oxygenators beweglich in einem Rahmen gehalten sind, ist es vorteilhaft, wenn der Rahmen relativ zum Gehäuse beweglich gehalten ist. Dadurch sind letztlich die Matten relativ zum Gehäuse beweglich, wodurch eine Abfederung einer kurzzeitigen Druckspitze erzielt werden kann.

Insbesondere die Kombination der Variation des Strömungsvolumenstroms über der Zeit mit einer gegebenenfalls auch nur geringen Abfederung für Volumenstromspitzen führt zu einer effektiven die Matten schonenden Durchströmung.

Ein kompakter Aufbau und eine schonende Behandlung des Blutes wird ferner dadurch erzielt, dass eine Verbindungsleitung zwischen der Blutpumpe und dem Oxygenator weniger als 20 cm, vorzugsweise weniger als 15 cm und besonders bevorzugt weniger als 5 cm lang ist. Die Blutpumpe und der Oxygenator werden somit möglichst nahe aneinander angeordnet und vorzugsweise sogar in demselben Gehäuse gehalten.

Weitere Vorteile ergeben sich durch unterschiedliche Ausbildungen der Matten und insbesondere durch die Anordnung der Matten relativ zueinander. Somit ist zunächst vorgesehen, dass die Matten von einer zur nächsten Ebene in einem Winkel von 90 ° zueinander angeordnet sind. Der Winkel beschreibt den Winkel zwischen den Hauptdurchströmungsrichtungen jeweils übereinander gelagerter Matten.

Weiterhin wird vorgeschlagen, dass die Matten rechteckig und vorzugsweise quadratisch ausgebildet sind.

Eine weitere Ausführungsform sieht vor, dass der Oxygenator ein zylinderförmiges Gehäuse aufweist, in dem die Matten parallel zu einer orthogonalen Schnittkreisfläche des zylinderförmigen Gehäuses angeordnet sind.

Ein einfacher Aufbau ergibt sich dadurch, dass der Oxygenator einen zentralen Zugang und vorzugsweise auch einen zentralen Ausgang aufweist. Alternativ kann der Oxygenator auch einen dezentralen Zugang und auch einen dezentralen Ausgang aufweisen.

Eine besonders bevorzugte Ausführungsvariante sieht vor, dass der Oxygenator einen Luftblasensensor aufweist. Dies ermöglicht es, direkt am Oxygenator im System befindliches Gas zu ermitteln, um gegebenenfalls durch eine Variation des Strömungsvolumens, beispielsweise durch Umschaltung auf pulsatile Anströmung, das Gas aus dem Oxygenator zu treiben.

Vorteilhafte Ausführungsvarianten ergeben sich je nach Einsatzzweck, indem der Oxygenator der Pumpe nachgeschaltet ist oder die Pumpe dem Oxygenator nachgeschaltet wird.

Vorteilhafte Ausführungsformen sind in der Zeichnung dargestellt und werden im Folgenden näher erläutert. Es zeigt
- Figur 1: schematisch einen runden Oxygenator mit zentralem Anschluss für den Zu- und Ablauf,
- Figur 2: schematisch einen runden Oxygenator mit dezentralem Zu- und Ablauf,
- Figur 3: schematisch einen eckigen Oxygenator mit dezentralem Zu- und Ablauf,
- Figur 4: schematisch einen runden Oxygenator mit zentralem Anschluss und einer Steuerung,
- Figur 5: schematisch einen runden Oxygenator mit dezentralem Anschluss,
- Figur 6: schematisch einen eckigen Oxygenator mit zentralem Anschluss,
- Figur 7: schematisch einen Schnitt in einer zur Rotorachse der Pumpe orthogonalen Schnittebene unterhalb des Rotors der Pumpe ohne Magnete,
- Figur 8: schematisch den in Figur 7 gezeigten Schnitt mit Magneten und
- Figur 9: schematisch einen runden Oxygenator mit zentralem Anschluss mit querschnittsvergrößendem Blutzulauf und Ausgleichsgefäß.

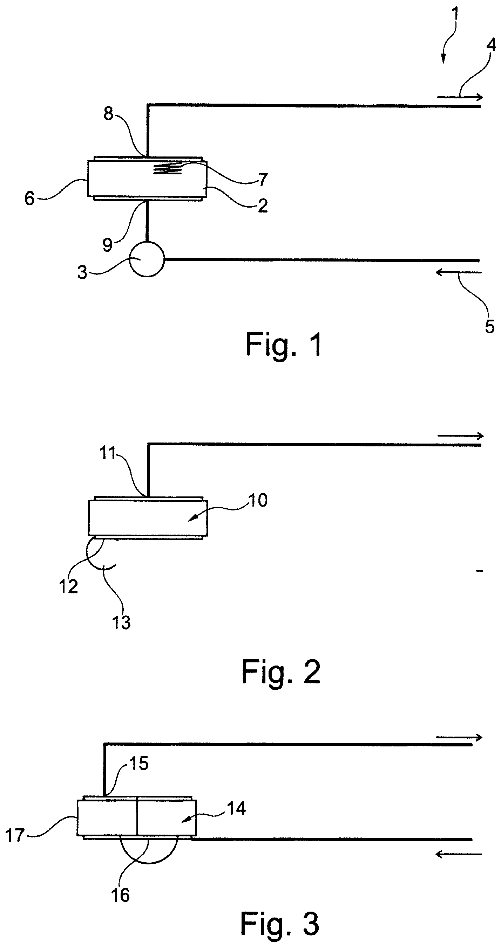

Das in Figur 1 gezeigte System 1 besteht aus einem runden Oxygenator 2 und einer Pumpe 3. Die mit den Pfeilen 4 und 5 angedeutete Flussrichtung zeigt, dass zunächst die Pumpe 3 und anschließend der Oxygenator 2 durchströmt werden. Der Oxygenator 2 hat ein Gehäuse 6, in dem schematisch angedeutete Matten 7 gestapelt sind, die Hohlfasern aufweisen.

Der Oxygenator 2 weist als ersten Anschluss einen zentralen Zugang 9 und als zweiten Anschluss einen zentralen Ausgang 8 auf.

Die Figur 2 zeigt einen ähnlichen Aufbau, bei dem der Oxygenator 10 einen dezentralen Zugang 12, der direkt mit der Pumpe 13 in Verbindung steht, und einen dezentralen Ausgang 11 aufweist.

Die Figur 3 zeigt einen weiteren Oxygenator 14, der einen dezentralen Zugang 16 und einen dezentralen Ausgang 15 aufweist und dessen Gehäuse 17 quadratisch ausgebildet ist.

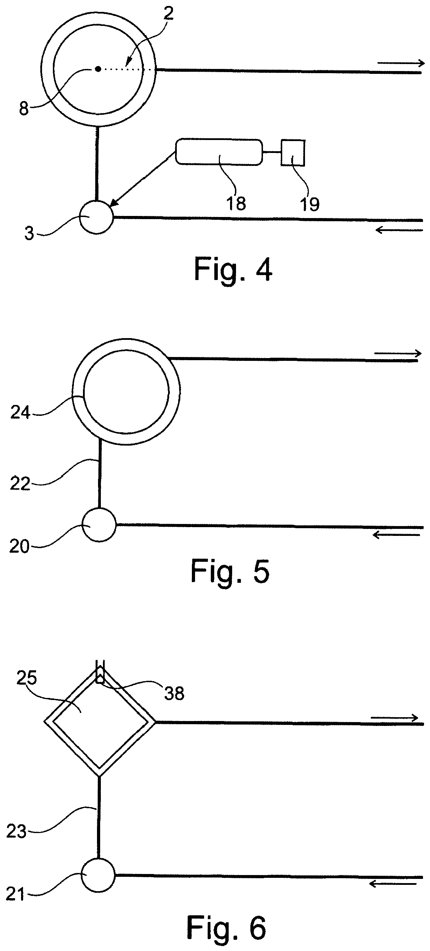

Die Figur 4 zeigt den in Figur 1 gezeigten runden Oxygenator 2 in einer Draufsicht mit dem verdeckten zentralen Anschluss 8 und der Blutpumpe 3, die mit einer Steuerung 18 in Verbindung steht, die eine kontinuierliche Variation des Strömungsvolumens über der Zeit ermöglicht. Die Steuerung 18 steht mit dem schematisch angedeuteten EKG 19 in Verbindung.

Die Figuren 5 und 6 zeigen jeweils eine Ansicht der Anordnungen gemäß den Figuren 2 und 3 in schematisch ähnlichem Aufbau - jedoch mit einer Blutpumpe 20 bzw. 21, die durch eine Leitung 22 bzw. 23 mit dem zylinderförmigen Oxygenator 24 bzw. dem quadratischen Oxygenator 25 in Verbindung steht.

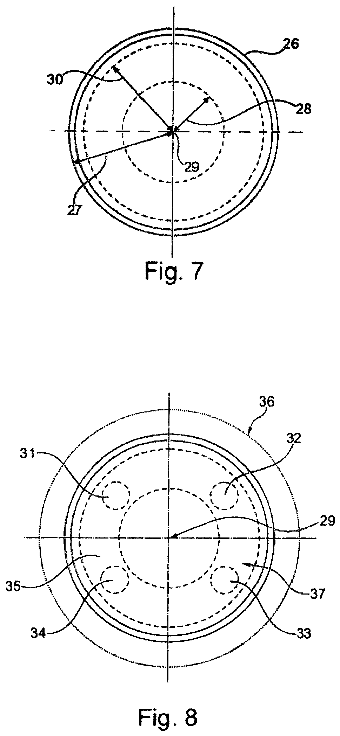

Die Figur 7 zeigt die Unterseite eines Rotors 26, der einen äußeren Durchmesser 27 von etwa 2,5 oder 3 cm aufweist. Der kleinere mittlere radiale Abstand der Magnete von der Achse 29 mit 5 mm ist mit dem Pfeil 28 angedeutet und der größere mittlere radiale Abstand mit 10 mm ist mit dem Pfeil 30 angedeutet.

Mehrere Magnete 31, 32, 33 und 34 sind als kleine, runde Magnetstücke in dem Ring 35 angeordnet. Als Magnete können entweder 4 bis 8 kleine Magnetstücke oder ein Ringmagnet verwendet werden. Diese sind konzentrisch um die Achse 29 innerhalb eines Gehäuses 36 angeordnet und bilden ein Magnetsystem 37, mit dem die Kraft eines Antriebs (nicht gezeigt) auf den Rotor 26 übertragen wird.

In Figur 6 ist ein Oxygenator 25 mit einem Luftblasensensor 38 schematisch angedeutet.

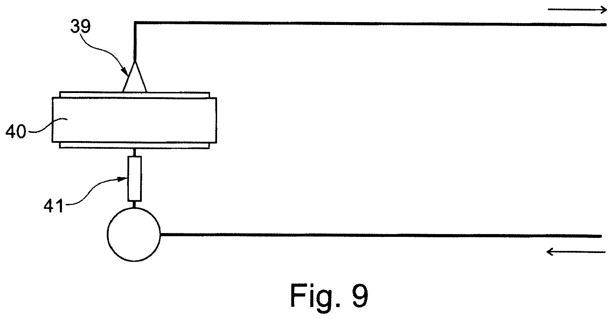

Die Figur 9 zeigt einen Ablauf 39 vom Oxygenator 40, der sich kurz hinter dem Oxygenator trichterförmig aufweitet, sodass das Blut die Matten im Oxygenator mit einer geringeren Geschwindigkeit anströmt als in der Leitung hinter dem Oxygenator.. Entsprechend kann auch in Flussrichtung vor dem Oxygenator ein Zulauf vorgesehen sein, der sich trichterförmig aufweitet, um die angeströmten Platten zu schonen. Ein derartiger trichterförmiger Zulauf oder Ablauf ist für jede Art von Oxygenator geeignet, um im Oxygenator die Flussgeschwindigkeit zu reduzieren und damit die angeströmten Platten zu schonen.

Außerdem zeigt die Figur 9 ein Ausgleichsgefäß 41, in dem ein Gaspolster vorgesehen ist, um einen schwankenden Druck im System abzufedern. Ein derartiges Ausgleichsgefäß 41 kann an jeder Stelle des Systems und vorzugsweise in der Nähe des Oxygenators angeordnet werden.

Die Figuren zeigen eine Durchströmung des Systems, beider das Blut zuerst die Blutpumpe und dann den Oxygenator durchströmt. Das System kann aber auch in der entgegengesetzten Richtung durchströmt werden, sodass das Blut zuerst den Oxygenator und erst danach die Blutpumpe durchströmt.

### Gegenstände

1. System für die extrakorporale Membranoxygenierung (1) mit einer Blutpumpe (3, 13) und einem Oxygenator (2, 10), wobei die Blutpumpe (3, 13) eine Steuerung (18) aufweist, die eine kontinuierliche Variation des Strömungsvolumens über der Zeit ermöglicht ***dadurch gekennzeichnet, dass*** der Oxygenator (2, 10) in einem Gehäuse (6) gestapelte Matten (7) mit Fasern aufweist, die parallel zueinander angeordnet sind.
2. System nach Gegenstand 1, ***dadurch gekennzeichnet, dass*** die Steuerung (18) mit einem EKG (19) in Verbindung steht.
3. System insbesondere nach Gegenstand 1 oder 2, ***dadurch gekennzeichnet, dass*** die Blutpumpe (3, 13) einen Rotor (26) aufweist, dessen äußerer Durchmesser (27) kleiner als 4 cm, bevorzugt kleiner als 3,5 cm ist.
4. System nach einem der vorhergehenden Gegenstände ***dadurch gekennzeichnet, dass*** die Blutpumpe (3, 13) einen Rotor (26) aufweist, dessen Massenträgheitsmoment kleiner als 5000 g/mm² und vorzugsweise kleiner als 1000 g/mm² ist.
5. System nach einem der vorhergehenden Gegenstände, ***dadurch gekennzeichnet, dass*** die Blutpumpe (3, 13) einen Rotor (26) aufweist, der über Magnete (31 bis 34) mit einem Antrieb in Verbindung steht, der den Rotor (26) um eine Achse (29) dreht, wobei die Magnete (31 bis 34) in einem mittleren radialen Abstand (28, 30) von 5 bis 10 mm von der Achse (29) entfernt angeordnet sind.
6. System nach einem der vorhergehenden Gegenstände ***dadurch gekennzeichnet, dass*** die Blutpumpe (3, 13) einen Rotor (26) aufweist, der einen axialen Strömungsanteil bewirkt.
7. System nach einem der vorhergehenden Gegenstände ***dadurch gekennzeichnet, dass*** zwischen Blutpumpe (3, 13) und Oxygenator (2, 10) eine Druckabfederung angeordnet ist.
8. System nach Gegenstand 7, ***dadurch gekennzeichnet, dass*** die Druckabfederung ein Ausgleichsgefäß (41) mit einem Gaspolster aufweist.
9. System nach Gegenstand 7, ***dadurch gekennzeichnet, dass*** die Druckabfederung eine Leitung mit mindestens einem flexiblen Wandbereich aufweist.
10. System nach einem der vorhergehenden Gegenstände ***dadurch gekennzeichnet, dass*** eine Verbindungsleitung (22, 23) zwischen der Blutpumpe (3, 13) und dem Oxygenator (2, 10) weniger als 20 cm, vorzugsweise weniger als 15 cm und besonders bevorzugt weniger als 5 cm lang ist.
11. System nach einem der vorhergehenden Gegenstände, ***dadurch gekennzeichnet, dass*** der Querschnitt des Blutzulaufs (39) vor der angeströmten Matte vergrößert ist, um die Strömungsgeschwindigkeit des Blutes zu reduzieren.
12. System nach einem der vorhergehenden Gegenstände ***dadurch gekennzeichnet, dass*** die Matten des Oxygenators beweglich in einem Rahmen gehalten sind.
13. System nach Gegenstand 12, ***dadurch gekennzeichnet, dass*** der Rahmen relativ zum Gehäuse beweglich gehalten ist.
14. System nach einem der vorhergehenden Gegenstände, ***dadurch gekennzeichnet, dass*** die Matten von einer zur nächsten Ebene in einem Winkel von 90 ° zueinander angeordnet sind.
15. System nach einem der vorhergehenden Gegenstände, ***dadurch gekennzeichnet, dass*** die Matten rechteckig und vorzugsweise quadratisch ausgebildet sind.
16. System nach einem der vorhergehenden Gegenstände, ***dadurch gekennzeichnet, dass*** der Oxygenator (2, 10) ein zylinderförmiges Gehäuse aufweist, in dem die Matten parallel zu einer Schnittkreisfläche angeordnet sind.
17. System nach einem der vorhergehenden Gegenstände, ***dadurch gekennzeichnet, dass*** der Oxygenator (10) einen dezentralen Zugang (12) und vorzugsweise auch einen dezentralen Ausgang (11) aufweist.
18. System nach einem der vorhergehenden Gegenstände, ***dadurch gekennzeichnet, dass*** der Oxygenator (2) einen zentralen Zugang (9) und vorzugsweise auch einen zentralen Ausgang (8) aufweist.
19. System nach einem der vorhergehenden Gegenstände:, ***dadurch gekennzeichnet, dass*** der Oxygenator (25) einen Luftblasensensor (38) aufweist.
20. System nach einem der vorhergehenden Gegenstände, ***dadurch gekennzeichnet, dass*** der Oxygenator (3, 13) der Blutpumpe (3, 13) nachgeschaltet ist.
21. System nach einem der vorhergehenden Gegenstände ***dadurch gekennzeichnet, dass*** die Blutpumpe dem Oxygenator nachgeschaltet ist.

## Patentansprüche

1. System für die extrakorporale Membranoxygenierung (1) mit einer Blutpumpe (3, 13) und einem Oxygenator (2, 10), wobei die Blutpumpe (3, 13) eine Steuerung (18) aufweist, die eine kontinuierliche Variation des Strömungsvolumens über der Zeit ermöglicht ***dadurch gekennzeichnet, dass** der* Oxygenator (2, 10) in einem Gehäuse (6) gestapelte Matten (7) mit Fasern aufweist, die parallel zueinander angeordnet sind.

2. System nach Anspruch 1, ***dadurch gekennzeichnet, dass*** die Steuerung (18) mit einem EKG (19) in Verbindung steht.

3. System insbesondere nach Anspruch 1 oder 2, ***dadurch gekennzeichnet, dass** die* Blutpumpe (3, 13) einen Rotor (26) aufweist, dessen äußerer Durchmesser (27) kleiner als 4 cm, bevorzugt kleiner als 3,5 cm ist.

4. System nach einem der vorhergehenden Ansprüche, ***dadurch gekennzeichnet, dass*** die Blutpumpe (3, 13) einen Rotor (26) aufweist, dessen Massenträgheitsmoment kleiner als 5000 g/mm² und vorzugsweise kleiner als 1000 g/mm² ist.

5. System nach einem der vorhergehenden Ansprüche, ***dadurch gekennzeichnet, dass*** die Blutpumpe (3, 13) einen Rotor (26) aufweist, der über Magnete (31 bis 34) mit einem Antrieb in Verbindung steht, der den Rotor (26) um eine Achse (29) dreht, wobei die Magnete (31 bis 34) in einem mittleren radialen Abstand (28, 30) von 5 bis 10 mm von der Achse (29) entfernt angeordnet sind.

6. System nach einem der vorhergehenden Ansprüche, ***dadurch gekennzeichnet, dass*** die Blutpumpe (3, 13) einen Rotor (26) aufweist, der einen axialen Strömungsanteil bewirkt.

7. System nach einem der vorhergehenden Ansprüche, ***dadurch gekennzeichnet, dass*** zwischen Blutpumpe (3, 13) und Oxygenator (2, 10) eine Druckabfederung angeordnet ist.

8. System nach einem der vorhergehenden Ansprüche, ***dadurch gekennzeichnet, dass*** eine Verbindungsleitung (22, 23) zwischen der Blutpumpe (3, 13) und dem Oxygenator (2, 10) weniger als 20 cm, vorzugsweise weniger als 15 cm und besonders bevorzugt weniger als 5 cm lang ist.

9. System nach einem der vorhergehenden Ansprüche, ***dadurch gekennzeichnet, dass*** der Querschnitt des Blutzulaufs (39) vor der angeströmten Matte vergrößert ist, um die Strömungsgeschwindigkeit des Blutes zu reduzieren.

10. System nach einem der vorhergehenden Ansprüche, ***dadurch gekennzeichnet, dass*** die Matten des Oxygenators beweglich in einem Rahmen gehalten sind.

11. System nach Anspruch 10, ***dadurch gekennzeichnet, dass*** der Rahmen relativ zum Gehäuse beweglich gehalten ist.

12. System nach einem der vorhergehenden Ansprüche, ***dadurch gekennzeichnet, dass*** die Matten von einer zur nächsten Ebene in einem Winkel von 90° zueinander angeordnet sind.

13. System nach einem der vorhergehenden Ansprüche, ***dadurch gekennzeichnet, dass*** die Matten rechteckig und vorzugsweise quadratisch ausgebildet sind.

14. System nach einem der vorhergehenden Ansprüche, ***dadurch gekennzeichnet, dass*** der Oxygenator (2, 10) ein zylinderförmiges Gehäuse aufweist, in dem die Matten parallel zu einer Schnittkreisfläche angeordnet sind.

15. System nach einem der vorhergehenden Ansprüche, ***dadurch gekennzeichnet, dass*** der Oxygenator (25) einen Luftblasensensor (38) aufweist.
